(19) Europäisches Patentamt
European Patent Office
Office européen des brevets

(11) EP 3 325 961 B1

# (12) EUROPÄISCHE PATENTSCHRIFT

(45) Veröffentlichungstag und Bekanntmachung des Hinweises auf die Patenterteilung:
**30.07.2025 Patentblatt 2025/31**

(21) Anmeldenummer: **16734579.2**

(22) Anmeldetag: **16.06.2016**

(51) Internationale Patentklassifikation (IPC):
***G01N 29/024** (2006.01)* ***G01N 29/032** (2006.01)*
***G01N 29/07** (2006.01)* ***G01N 29/11** (2006.01)*
***G01N 29/30** (2006.01)* ***G01N 29/46** (2006.01)*

(52) Gemeinsame Patentklassifikation (CPC):
**G01N 29/024; G01N 29/032; G01N 29/07; G01N 29/11; G01N 29/30; G01N 29/46; G01N 33/2847;** G01N 2291/0226; G01N 2291/045; G01N 2291/048

(86) Internationale Anmeldenummer:
**PCT/EP2016/063868**

(87) Internationale Veröffentlichungsnummer:
**WO 2017/012794 (26.01.2017 Gazette 2017/04)**

## (54) VORRICHTUNG UND VERFAHREN ZUR UNTERSUCHUNG VON MATERIALIEN DURCH AKUSTISCHE SPEKTROSKOPIE

DEVICE AND METHOD FOR EXAMINING MATERIALS BY MEANS OF ACOUSTIC SPECTROSCOPY

DISPOSITIF ET PROCÉDÉ POUR ANALYSER DES MATÉRIAUX PAR SPECTROSCOPIE ACOUSTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.07.2015 EP 15177198**
**26.10.2015 DE 202015105692 U**
**08.02.2016 DE 102016102131**

(43) Veröffentlichungstag der Anmeldung:
**30.05.2018 Patentblatt 2018/22**

(73) Patentinhaber: **Passerro Verwaltungs- und Beteiligungsgesellschaft mbH**
**04299 Leipzig (DE)**

(72) Erfinder:
- **FOKOW, Krzysztof**
  **01-496 Warszawa (PL)**
- **WROBEL, Miroslaw**
  **97753 Karlstadt (DE)**

(74) Vertreter: **advotec.**
**Patent- und Rechtsanwaltspartnerschaft mbB**
**Beethovenstraße 5**
**97080 Würzburg (DE)**

(56) Entgegenhaltungen:
**DE-A1- 102008 014 300** **DE-U1- 202007 017 911**
**US-A- 5 433 112** **US-A1- 2006 028 096**
**US-A1- 2015 059 442**

- **JU BING-FENG ET AL: "Simultaneous measurement of local longitudinal and transverse wave velocities, attenuation, density, and thickness of films by using point-focus ultrasonic spectroscopy", JOURNAL OF APPLIED PHYSICS, AMERICAN INSTITUTE OF PHYSICS, US, vol. 112, no. 8, 15 October 2012 (2012-10-15), pages 84910 - 84910, XP012167758, ISSN: 0021-8979, [retrieved on 20121025], DOI: 10.1063/1.4758136**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 325 961 B1

## Beschreibung

Die Erfindung betrifft eine Vorrichtung und ein Verfahren zur Untersuchung von Materialen durch akustische Spektroskopie gemäß den unabhängigen Hauptansprüchen 1 bzw. 8.

Ein Verfahren zur akustischen Spektroskopie von Glasmaterialien ist beispielsweise aus der US 2006/028096 A1 bekannt. Gemäß diesem Verfahren erfolgt die Berechnung der Dämpfung und der Ultraschallgeschwindigkeit auf Basis der Messung von Amplitude und Phasenverschiebung, wodurch auf die Materialeigenschaften des Glases geschlossen werden kann. Ebenso offenbart die DE 10 2008 014300 A1 ein gattungsgemäßes Verfahren zur Bestimmung des Zustands eines Stoffgemisches mittels Messung der Schallgeschwindigkeit und/oder Ultraschalldämpfung. Um aus Schallgeschwindigkeit und Dämpfung Rückschlüsse auf die Zusammensetzung des zu untersuchenden Stoffgemischs zu ziehen, werden verfahrensgemäß die Amplituden der transmittierten und reflektierten Schallwellen ausgewertet und die Laufzeit der einzelnen Schallwellen aus der quadrierten Signalamplitude der gesendeten und empfangenen Signale berechnet.

Ju Bing-Feng et al. schlagen in ihrer Veröffentlichung mit dem Titel "Simultaneous measurement of longitudinal and transverse velocities, attenuation, density,. and thickness of films by using point-focus ultrasonic spectroscopy" im Journal of Applied Physics ein weiteres gattungsgemäßes Verfahren zur Bestimmung der akustischen und geometrischen Eigenschaften eines dünnschichtigen Prüfmaterials mittels Spektroskopie vor, bei dem die akustische Impedanz, der Dämpfungskoeffizient und die Durchlaufzeit der fokussierten Ultraschallwellen aus den Reflektionsspektren der Ultraschallwellen berechnet werden.

Zur kontinuierlichen Untersuchung eines Mediums ist aus der DE 20 2007 017911 U1 eine gattungsgemäße Vorrichtung bekannt, die eine kegelförmige Abstrahlcharakteristik erzeugt, um anhand des Vergleichs eines Referenzmusters von Phasenverschiebungen mit den an einem Empfänger auftretenden Phasenverschiebungen eine Zustandsbeurteilung des Prüfmaterials zu ermöglichen.

Aus der US 5 433 112 A ist ein weiteres gattungsgemäßes Verfahren und eine Vorrichtung zur Charakterisierung einer Polymerschmelze bekannt, mit dem bzw. der die Zeitverschiebung zwischen zwei aufeinanderfolgenden aus einer Polymerschmelze austretenden Ultraschallechos ermittelt werden kann, wobei die Messstrecke zwischen einem Ultraschallsender und einem Ultraschallempfänger aus zwei Pufferstäben und der mittig angeordneten Polymerschmelze gebildet ist. Zur Berechnung der akustischen Materialkennwerte werden bei diesem Verfahren die auftretenden Amplitudenänderungen einbezogen.

Die US 2015/0059442 A1 schlägt ein Verfahren zur akustischen Spektroskopie unter Aussendung von Schallwellen unterhalb des Ultraschallfrequenzbereichs vor. Die Auswertung der reflektierten akustischen Spektren nach Interaktion mit der zu charakterisierenden Suspension dient der Bestimmung der Eigenschaften, des Gehalts und/oder der Größe der Partikel in der Suspension.

Eine weitere Vorrichtung zur Untersuchung von flüssigen oder gasförmigen Medien durch akustische Spektroskopie ist beispielsweise aus der DE 103 24 990 B2 bekannt. Bei dieser Vorrichtung sind eine Sendeeinrichtung zum Senden mehrerer Sendesignale unterschiedlicher Frequenz und eine Empfangseinrichtung zum Empfangen entsprechender Empfangssignale vorgesehen. Mit einer Verarbeitungseinrichtung kann dann die Phasenverschiebung zu jedem Sende- und Empfangssignalpaar ermittelt und daraus ein das untersuchte Medium qualifizierender Wert anhand der Phasenverschiebung abgeleitet werden. Bei der dort beschriebenen Vorrichtung werden nacheinander in unmittelbarer Abfolge Signalpakete unterschiedlicher Frequenzen gesendet. Die Frequenzen bewegen sich vorzugsweise im Bereich zwischen 1 und 15 MHz, wobei jedes Signalpaket vorzugsweise wenigstens 100 Perioden umfassen sollte. Zu jedem Signalpaket und damit zu jeder Frequenzstufe werden über die Empfangseinrichtung die spezifischen Empfangssignale aufgenommen und danach einer Verarbeitungseinrichtung zugeleitet. In der Verarbeitungseinrichtung wird zu jedem frequenzspezifischen Sende- und Empfangssignalpaar der durch den Durchtritt durch das untersuchte Medium verursachte Phasenverschiebungswinkel zwischen den beiden Signalen ermittelt. So wird im Endeffekt durch diese frequenzspezifische Abtastung des Mediums eine Vielzahl unterschiedlicher frequenzspezifischer Phasenverschiebungswerte erfasst, deren Anzahl abhängig von der Anzahl der Sende- und Empfangssignalpaare ist. Auf diese Weise erhält man Informationen über das Verhalten des Mediums aus einem sehr großen Frequenzbereich definiert über einzelne Frequenzstufen, wobei diese Informationen Aussagen über die Eigenschaften des untersuchten Mediums ermöglichen. Durch die frequenzspezifische Abtastung des Mediums und die entsprechende Bestimmung der frequenzbezogenen Phasenwinkel lässt sich nämlich eine wesentlich spezifischere Untersuchung des Mediums erreichen, da sich eine Änderung des Mediums unterschiedlich auf das jeweilige frequenzspezifische Sende- und Empfangssignalpaar auswirkt.

Nachteilig an der in der DE 103 24 990 B2 beschriebenen Vorrichtung ist es, dass sie auf der Auswertung der Phasenverschiebungswerte beruht, die durch den Durchtritt des Sendesignals durch das zu untersuchende Merkmal verursacht und durch das Empfangssignal dokumentiert wird. Denn die Auswertung der Phasenverschiebung zwischen Sendesignal und Empfangssignal kann zu unerwünschten Informationsverlusten führen, durch die das Auswertungsergebnis signifikant verfälscht wird. Diese Informationsverluste beruhen darauf, dass der Wert der Phasenverschiebung auf einen Wertebereich zwischen 0 und 360° begrenzt ist. Ist die Phasenverschiebung zwischen Sendesignal und Empfangssignal jedoch größer als 360°, so liefert das in der Druckschrift beschriebene Verfahren einen Phasenverschiebungswert, der nicht eindeutig zugeordnet werden kann. Beträgt die Phasenverschiebung zwischen Sendesignal und Empfangssignal beispielsweise 400°, so liefert die in der Druckschrift beschriebene Auswertevorrichtung einen Phasenverschiebungswert von 40°. Die daraus abgeleiteten Auswertungsergebnisse sind somit entscheidend verfälscht und unbrauchbar, da die Auswertung Ergebnisse liefert, die einer Phasenverschiebung von 40° entspricht und nicht einer Phasenverschiebung von 400° (= 360° + 40°).

Zur Lösung dieses Problems der nicht eindeutig zuordenbaren Ergebnisse bei der Auswertung der Phasenverschiebung zwischen Sendesignal und Empfangssignal schlägt die DE 198 41 154 A1 die Generierung von Sendesignalen mit unterschiedlicher Frequenz vor. Die verschiedenen Frequenzen dienen dabei in der Art eines Nonius bei der Bewertung der Phasenverschiebungen. So ist es möglich, die gemessenen Phasenverschiebungswerte eindeutig der tatsächlichen Phasenverschiebung zwischen Sendesignal und Empfangssignal zuzuordnen. Dieses Verfahren hat jedoch den Nachteil, dass die Schallwellen in dem zu untersuchenden Medium abhängig von der jeweiligen Sendefrequenz mit unterschiedlicher Schallgeschwindigkeit übertragen werden, wobei diese verschiedenen Übertragungsgeschwindigkeiten im zu untersuchenden Medium zu erheblichen Messfehlern führen können. Die Abhängigkeit der Ausbreitungsgeschwindigkeit des Sendesignals von der jeweiligen Sendefrequenz wird durch die Kramers-Kronig-Gleichung beschrieben. Insofern hat sich das Messverfahren gemäß der DE 198 41 154 A1 als ungeeignet erwiesen, da die Messfehler aufgrund der nicht einheitlichen Ausbreitungsgeschwindigkeit der Schallwellen abhängig von der jeweiligen Sendefrequenz nicht berücksichtigt werden können.

Die Grundlagen der akustischen Spektroskopie, die der erfindungsgemäßen Vorrichtung zugrunde liegen, sind beispielsweise, jedoch keineswegs ausschließlich in folgenden Fachbüchern beschrieben:

Fachbuch 1: Molekularakustik, Werner Schaaffs, Springer Verlag, 1963 (ISBN-10:3642491413, ISBN-13:978-3642491412)

Fachbuch 2: Molecular Acustics/Molekularakustik, K.-H. Hellwege, A.M. Hellwege, W. Schaaffs, Springer Verlag, 1967 (ISBN-10:3540038973, ISBN-13:978-3540038979)

Fachbuch 3: Molecular Acustics, A.J. Matheson, John Willy & Sons Verlag, 1971 (ISBN-10:1861561857, ISBN-13:978-1861561855)

Fachbuch 4: Werkstoffprüfung mit Ultraschall, Josef Krautkrämer, Herbert Krautkrämer, Springer-Verlag, 1975

Ausgehend von diesem Stand der Technik soll eine Vorrichtung und ein Verfahren zur Untersuchung von Prüfmaterialien durch akustische Spektroskopie vorgeschlagen werden. Insbesondere soll ein Verfahren und eine Vorrichtung vorgeschlagen werden, mit denen die Werkstoffeigenschaften von Transformatorenölen online untersucht werden können.

Transformatoren dienen der Wandlung einer Eingangs-Wechselspannung in eine Ausgangs-Wechselspannung, wobei die elektrische Energie durch magnetische Felder zwischen zwei Spulen übertragen werden muss. Bei sehr hohen Eingangs- bzw. Ausgangs-Wechselspannungen werden üblicherweise Transformatoren eingesetzt, deren Gehäuse mit Transformatorenöl gefüllt ist, um für eine ausreichende Isolation und Kühlung zwischen den Übertragungsspulen zu sorgen. Da das Transformatorenöl stark hygroskopisch ist, neigt es dazu im Laufe der Zeit Wasser aufzunehmen, das dann zu Säuren im Transformatorenöl umgebaut wird. Durch das Wasser bzw. die Säure im Transformatorenöl wird die Funktionalität des Transformators im Laufe der Zeit zunehmend eingeschränkt, da durch das Wasser bzw. die Säure die vom Transformatorenöl gewährleistete elektrische Isolierung, die beispielsweise in der Art der maximalen Durchschlagsspannung angegeben werden kann, herabgesetzt wird. Bei bekannten Transformatoren ist es deshalb üblich das Transformatorenöl in regelmäßigen Wartungszyklen auszuwechseln oder zu regenerieren, um die Funktionalität des Transformators zuverlässig zu gewährleisten. Diese regelmäßige Auswechslung des Transformatorenöls ist jedoch außerordentlich aufwendig und teuer, da das Transformatorenöl stark giftige Inhaltsstoffe beinhaltet und deshalb aufwendig entsorgt bzw. wiederaufbereitet werden muss. Außerdem wenn die Wartungszyklen zu lang gewählt werden, um die Kosten für die Auswechslung des Transformatorenöls möglichst gering zu halten, kann dies zu einem Totalversagen des Transformators führen.

Bekannte Verfahren zur Untersuchung des Transformatorenöls setzen voraus, dass der Transformator geöffnet und eine Probe des Transformatorenöls entnommen werden muss. Das Öffnen des Transformators zur Probenentnahme ist jedoch hoch problematisch, da das Transformatorenöl stark hygroskopisch ist und beim Öffnen des Transformatorgehäuses aus der Umgebungsluft eine relativ große Menge von Wassermolekülen in das Transformatorenöl übergeht.

Aufgabe der vorliegenden Erfindung ist es deshalb, eine Vorrichtung und ein Verfahren zur Untersuchung von Materialien durch akustische Spektroskopie vorzuschlagen, bei dem die oben benannten Nachteile vermieden werden.

Diese Aufgabe wird durch eine Vorrichtung und ein Verfahren nach der Lehre der unabhängigen Hauptansprüche 1 bzw. 8 gelöst.

Vorteilhafte Ausführungsformen der Erfindung sind Gegenstand der Unteransprüche.

Die erfindungsgemäße Vorrichtung umfasst eine Messstrecke, die aus einem Referenzmaterial und dem eigentlich zu untersuchenden Prüfmaterial besteht. Ziel der Untersuchung ist es dabei, die akustischen Materialkennwerte des Prüfmaterials zu ermitteln. Die akustischen Materialkennwerte des Referenzmaterials für unterschiedliche Frequenzen, d.h. die Dichte, die Schallübertragungsgeschwindigkeit, der akustische Dämpfungskoeffizient und/oder die akustische Impedanz des Referenzmaterials sind bekannt. Außerdem sind aus der Geometrie der Messstrecke die Länge des Referenzmaterials und die Länge des Prüfmaterials, die während der Untersuchung von den Ultraschallsignalen durchlaufen werden, bekannt.

Gattungsgemäß umfasst die Vorrichtung eine Ultraschall-Sendeeinrichtung zum Aussenden von Ultraschall-Sendesignalen mit einer Anfangsamplitude $A_0$ durch die Messstrecke. Weiter umfasst die Vorrichtung eine erste Ultraschall-Empfangseinrichtung, mit der die transmittierten Ultraschall-Empfangssignale nach Durchlaufen der Messstrecke empfangen und gemessen werden können. Neben der ersten Ultraschall-Empfangseinrichtung umfasst die Vorrichtung eine zweite Ultraschall-Empfangseinrichtung zum Empfang der an der Grenzfläche zwischen Prüfmaterial und Referenzmaterial reflektierten Ultraschall-Empfangssignale nach zweimaligem Durchlaufen des Referenzmaterials bzw. Prüfmaterials. Die zweite Ultraschall-Empfangseinrichtung kann gegebenenfalls auch durch entsprechendes Umschalten der Ultraschall-Sendeeinrichtung realisiert werden.

Sowohl die Sendeeinrichtung als auch die beiden Empfangseinrichtungen sind zum Senden bzw. zum Empfangen von Ultraschallsignalen unterschiedlicher Frequenz geeignet, um eine multispektrale Ultraschalluntersuchung des Prüfmaterials bei unterschiedlichen Sende- bzw. Empfangsfrequenzen durchführen zu können.

In Abkehr der Lehre aus der DE 103 24 990 B3 wird bei der Signalauswertung jedoch nicht die Phasenverschiebung zu jedem Sende- und Empfangssignalpaar bestimmt. Stattdessen wird erfindungsgemäß in einer ersten Verarbeitungseinrichtung für jedes Sendesignal die Durchlaufzeit des zugeordneten transmittierten Ultraschall-Empfangssignals nach vollständigem Durchlaufen der Messstrecke gemessen. Außerdem wird erfindungsgemäß mit einer zweiten Verarbeitungseinrichtung für jedes Sendesignal die Amplitude des zugeordneten, transmittierten Ultraschall-Empfangssignals nach Durchlaufen der Messstrecke ermittelt und mit einer dritten Verarbeitungseinrichtung für jedes Sendesignal die Amplitude des zugeordneten, des an der Grenzfläche zwischen Prüfmaterial und Referenzmaterial reflektierten Ultraschall-Empfangssignals, nach zweimaligem Durchlaufen des Referenzmaterials bzw. Prüfmaterials ermittelt.

Erfindungsgemäß werden aus den drei mittels der Verarbeitungseinrichtungen ermittelten Messwerten, nämlich der Durchlaufzeit, der Amplitude des transmittierten Ultraschall-Empfangssignals und/oder der Amplitude des reflektierten Ultraschall-Empfangssignals anschließend in einer Auswerteeinrichtung die akustischen Materialkennwerte des Prüfmaterials für unterschiedliche Frequenzen, d.h. die Dichte, die Schallübertragungsgeschwindigkeit, der akustische Dämpfungswert und/oder die akustische Impedanz des Prüfmaterials berechnet, wobei je nach akustischem Materialkennwert nicht alle ermittelten Messwerte in dessen Berechnung einfließen. Die Amplitude des transmittierten Ultraschall-Empfangssignals bzw. die Amplitude des reflektierten Ultraschall-Empfangssignals kann unmittelbar gemessen oder durch Bestimmung anderer Messwerte, beispielsweise der Signaldämpfung und anschließende Ableitung der Amplitudenwerte ermittelt werden.

Durch die unmittelbare Messung der Durchlaufzeit des transmittierten Ultraschall-Empfangssignals nach Durchlaufen der Messstrecke werden die aus dem Stand der Technik bekannten Messfehler, wie sie beispielsweise bei der Verwendung der Phasenverschiebung zwischen Sende- und Empfangssignal auftreten, erfindungsgemäß vermieden.

Untersuchungen an Prüfmaterialien, wie beispielsweise an Transformatorenölen, bei der Beaufschlagung mit Ultraschallsignalen und der anschließenden Ermittlung der Durchlaufzeit, der Amplitude des transmittierten Empfangssignals und der Amplitude des reflektierten Empfangssignals haben gezeigt, dass Veränderungen im Prüfmaterial sich signifikant in den Ergebnisdatensätzen aus Durchlaufzeit, Transmissions-amplitude und/oder Reflektionsamplitude abbilden. Die konventionelle akustische Spektroskopie, bei der bisher allein die Signaldämpfung gemessen wurde, wird also erfindungsgemäß dahingehend erweitert, dass nicht nur für jede Frequenz die spezifische und frequenzabhängige Dämpfung, d.h. das Verhältnis zwischen Eingangs-Amplitude und Transmissions-Amplitude bzw. das Verhältnis zwischen Eingangs-Amplitude und Reflektions-Amplitude, sondern auch die spezifische und frequenzabhängige Durchlaufzeit (Time of Flight/TOF) bestimmt wird. Durch die Messung der Durchlaufzeit kann nämlich die Dispersivität des untersuchten Materials ermittelt werden. Die Kombination der Messung von Dämpfung und Dispersivität des untersuchten Prüfmaterials lässt eine sehr differenzierte Charakterisierung des untersuchten Prüfmaterials zu.

Mit der erfindungsgemäßen Vorrichtung wird in Abkehr der bisherigen Untersuchungsmethoden nicht mehr die Phasenverschiebung, sondern die Durchlaufzeit des Ultraschallsignals selber gemessen. Um die Eigenschaften des untersuchten Materials mit einer ausreichenden Auflösung bestimmen zu können, ist es besonders vorteilhaft, wenn die Durchlaufzeit mit einer Auflösung von zumindest 100 Pikosekunden in der ersten Bearbeitungseinrichtung ermittelt werden kann. Bevorzugt sollte die Durchlaufzeit mit einer Auflösung von zumindest 10 Pikosekunden ermittelt werden können.

Welche Zeitmesseinrichtung in der ersten Verarbeitungseinrichtung zur Bestimmung der Durchlaufzeit vorgesehen ist, ist grundsätzlich beliebig. Um Auflösungen bei der Messung der Durchlaufzeit, insbesondere im Bereich von unter 100 Pikosekunden zu erreichen, ist es besonders vorteilhaft, wenn dazu so genannte Time-to-digital-Converter eingesetzt werden. Bei diesen Time-to-digital-Convertern handelt es sich um elektronische Baugruppen, die Zeitintervalle im Bereich von < 100 Pikosekunden messen und in eine digitale Ausgabe umwandeln können. Die Messung der Zeit beruht dabei auf der bekannten Durchlaufzeit eines elektrischen Signals durch die Baugruppen des TDC-Converters. Die Durchlaufzeit wird dabei dann dadurch bestimmt, wie viele der elektrischen Schaltkreise mit bekannter Durchlaufzeit zwischen der Abgabe des Sendesignals zum einen und dem Empfang des Eingangssignals zum anderen durchlaufen werden. Diese Anzahl der Durchläufe von elektronischen Baugruppen mit bekannter Durchlaufzeit wird dann gezählt, wobei die gemessene Durchlaufzeit dann der Multiplikation der bekannten Durchlaufzeit eines einzelnen Schaltkreises der Anzahl von Durchläufen entspricht.

Mit der zweiten Ultraschall-Empfangseinrichtung werden die Ultraschall-Empfangssignale empfangen, die nach Aussenden des Ultraschall-Sendesignals mit seiner Anfangsamplitude und nach Durchlaufen des Prüfmaterials bzw. Referenzmaterials zunächst an der Grenzfläche zwischen Prüfmaterial und Referenzmaterial reflektiert werden und anschließend ein zweites Mal das Prüfmaterial bzw. Referenzmaterial durchlaufen. Die geometrische Anordnung der zweiten Ultraschall-Empfangseinrichtung entspricht also exakt dem Teil der Messstrecke, an dem auch die Ultraschall-Sendeeinrichtung angeordnet ist. Zur Reduktion des gerätetechnischen Aufwands ist es deshalb besonders vorteilhaft, wenn die zweite Ultraschall-Empfangseinrichtung durch Umschalten der Ultraschall-Sendeeinrichtung realisiert wird. Dies bedeutet mit anderen Worten, dass bei Betrieb der Vorrichtung das Ultraschall-Sendesignal zunächst mit der Ultraschall-Sendeeinrichtung generiert und in die Messstrecke ausgesandt wird. Anschließend wird die Ultraschall-Sendeeinrichtung umgeschaltet, so dass sie als Ultraschall-Empfangseinrichtung arbeitet. Das Ultraschall-Sendesignal durchläuft dann zunächst ein erstes Mal das Referenzmaterial oder Prüfmaterial und wird anschließend an der Grenzfläche zwischen Prüfmaterial und Referenzmaterial reflektiert. Das reflektierte Ultraschall-Signal durchläuft dann zum zweiten Mal das Referenzmaterial und Prüfmaterial und gelangt zuletzt zurück an den Anfang der Messstrecke, wo es von der Ultraschall-Sendeeinrichtung, die umgeschaltet als Ultraschall-Empfangseinrichtung arbeitet, empfangen und gemessen werden kann.

Aus welchem Werkstoff das Referenzmaterial der Messstrecke hergestellt ist, ist grundsätzlich beliebig. Besonders vorteilhaft ist es, wenn das Referenzmaterial aus einem Festkörper, insbesondere aus Kunststoff oder Glas, besteht.

Mit der erfindungsgemäßen Vorrichtung können Transformatorenöle untersucht werden. Dazu wird zwischen der Grenzfläche des Referenzmaterials und der ersten Ultraschall-Empfangseinrichtung ein Hohlraum gebildet. Die Länge dieses Hohlraums zwischen der Grenzfläche einerseits und der Ultraschall-Empfangseinrichtung andererseits muss dabei exakt bestimmt sein. Während der eigentlichen Untersuchung wird dann dieser Hohlraum mit dem Prüfmaterial befüllt und die eigentliche Messung durchgeführt. Alternativ dazu ist es auch denkbar den Hohlraum während der Untersuchung von dem Prüfmaterial durchströmen zu lassen.

Zu welchem Zweck die erfindungsgemäße Vorrichtung eingesetzt wird, ist grundsätzlich beliebig. Besonders große Vorteile bietet die Vorrichtung bei der Online-Messung von Transformatorenöl, das regelmäßig ausgetauscht werden muss. Denn durch die Online-Messung des Öls kann der Austausch des Öls verschleißabhängig durchgeführt werden und die Nachteile von regelmäßig durchzuführenden Wartungsintervallen wären dabei vermieden. Insbesondere zur Beurteilung des Zustands von Transformatorenöl kann die erfindungsgemäße Vorrichtung sehr gut eingesetzt werden. Dazu steht die Vorrichtung mit einem Gefäß in Verbindung, in dem das zu beurteilende Transformatorenöl enthalten ist. Während der eigentlichen Untersuchung wird dann der Hohlraum zwischen der Grenzfläche des Referenzmaterials und der ersten Ultraschall-Empfangseinrichtung mit dem Öl aus dem Gefäß befüllt oder durchströmt. Durch die multispektrale Untersuchung des Öls werden dann die Dichte, die Schallübertragungsgeschwindigkeit, der akustische Dämpfungskoeffizient oder die akustische Impedanz des Öls ermittelt.

Soweit mit der erfindungsgemäßen Vorrichtung Transformatorenöl untersucht wird, ist es besonders vorteilhaft, wenn aus den akustischen Materialkennwerten des Transformatorenöls anschließend der Wassergehalt bzw. der Säuregehalt im Transformatorenöl abgeleitet wird. Denn diese beiden Kennwerte geben Auskunft über die Alterung des Transformatorenöls und können dahingehend benutzt werden, die Auswechslung des Transformatorenöls jeweils erst nach Erreichen eines bestimmten Alterungsgrads, gemessen am Wassergehalt bzw. Säuregehalt, durchzuführen.

Im Hinblick auf die besonders vorteilhafte Beurteilung von Transformatorenöl wird außerdem ein Transformator zur Wandlung einer Eingangswechselspannung in eine Ausgangswechselspannung vorgeschlagen, wobei der Transformator mit Transformatorenöl gefüllt ist und eine erfindungsgemäße Vorrichtung zur Online-Untersuchung des Transformatorenöls umfasst.

Das erfindungsgemäße Verfahren ist wiederum charakterisiert durch die Bestimmung der Durchlaufzeit des transmittierten Ultraschall-Empfangssignals, der Amplitude des transmittierten Ultraschall-Empfangssignals und der Amplitude des an der Grenzfläche zwischen Prüfmaterial und Referenzmaterial reflektierten Ultraschall-Empfangssignals. Mit diesen beiden Messwerten werden dann die gesuchten akustischen Materialkennwerte des Prüfmaterials für unterschiedliche Frequenzen, d.h. insbesondere die Dichte, die Schallübertragungsgeschwindigkeit, der akustische Dämpfungskoeffizient und/oder die akustische Impedanz, berechnet.

Welche Art von Ultraschallwellen zur Untersuchung mittels des erfindungsgemäßen Verfahrens bzw. der erfindungsgemäßen Vorrichtung eingesetzt werden, ist grundsätzlich beliebig. Besonders geeignet sind Longitudinal-Ultraschallwellen im unteren Megahertz-Bereich. Weiterhin ist es denkbar, dass die Ultraschallwellensignale unterschiedlicher Frequenz von der Ultraschall-Sendeeinrichtung einzeln, nacheinander oder als Gruppen nacheinander ausgesandt werden.

Alternativ dazu kann das Ultraschall-Sendesignal für die unterschiedlichen Frequenzen auch mittels der Ultraschall-Sendeeinrichtung auf ein gemeinsames Trägersignal aufmodelliert und ausgesendet werden. Auch ist es möglich, dass das Ultraschall-Sendesignal unterschiedlicher Frequenz und die Ultraschall-Sendeeinrichtung auf ein gemeinsames Trägersignal als Superposition aufaddiert und gemeinsam ausgesendet werden.

Nachfolgend soll die Erfindung anhand einer in den Zeichnungen schematisch dargestellten Ausführungsform beispielhaft erläutert werden.

Es zeigen:

**Fig. 1** eine erfindungsgemäße Vorrichtung mit ihren verschiedenen Funktionsmodulen als Prinzipskizze;

**Fig. 2** die Messstrecke der Vorrichtung gemäß Fig. 1 in einer vergrößerten Darstellung;

**Fig. 3** die Formelsammlung zur Berechnung der akustischen Materialkennwerte;

**Fig. 4** die Auswerteeinrichtung der Vorrichtung gemäß Fig. 1 mit den darin gespeicherten Formeln zur Berechnung der akustischen Materialkennwerte des zu untersuchenden Prüfmaterials.

**Fig. 1** zeigt in einer Prinzipskizze den prinzipiellen Aufbau einer erfindungsgemäßen Vorrichtung 01, wie sie zur Online-Untersuchung eines Prüfmaterials 02 eingesetzt werden kann. Bei dem Prüfmaterial 02 handelt es sich um Transformatorenöl 30 (siehe Fig. 2) aus einem Transformator 31. Durch Verbindungsleitungen 32 und 33 und durch Betrieb einer Pumpe 34 steht ein Hohlraum 35 in Verbindung mit dem Transformator 31, so dass das Transformatorenöl 30 aus dem Inneren des Gehäuses 36 im Umlauf durch den Hohlraum 35 gepumpt werden kann.

Den Aufbau der Messstrecke 37 bei Durchführung der eigentlichen Untersuchung des Prüfmaterials 02 soll nachfolgend kurz anhand der Skizze in Fig. 2 erläutert werden.

Die Messstrecke 37 besteht zum einen aus einem Referenzmaterial 38 und dem zu untersuchenden Prüfmaterial 02, bei dem es sich in der dargestellten Ausführungsform um das Transformatorenöl 30 handelt. Bei dem Referenzmaterial 38 kann es sich beispielsweise um einen Festkörper aus Glas oder Kunststoff handeln, wobei die akustischen Materialeigenschaften des Referenzmaterials 38, d.h. der akustische Dämpfungskoeffizient $\alpha_R$, die akustische Impedanz $Z_R$, die Dichte $\rho_R$, die Schallübertragungsgeschwindigkeit $c_R$ zumindest teilweise bekannt sein müssen. Außerdem ist die Länge $x_R$ des Referenzmaterials 38 und die Länge $x_M$ des Prüfmaterials, d.h. die lichte Weite des Hohlraums 35 in Signalrichtung bekannt.

Am Anfang der Messstrecke 07 befindet sich eine Ultraschall-Sendeeinrichtung 39, mit der Ultraschall-Sendesignale mit einer Anfangsamplitude $A_0$ generiert und in die Messstrecke eingekoppelt werden können. Bei der Ultraschall-Sendeeinrichtung 39 handelt es sich um ein kombiniertes Gerät, das durch Umschalten auch als Ultraschall-Empfangseinrichtung 40 arbeiten kann. Am Ende der Messstrecke 37 befindet sich ebenfalls eine Ultraschall-Empfangseinrichtung 41, mit der Ultraschallsignale empfangen werden können.

Bei der eigentlichen Untersuchung des Prüfmaterials 02 wird zunächst der Hohlraum 35 durch Betrieb der Pumpe 34 vom Transformatorenöl 30 durchströmt. Die Messungen des Transformatorenöls können dabei online ohne Öffnung des Gehäuses 36 erfolgen. Sobald der Hohlraum 35 vollständig mit Transformatorenöl 30 gefüllt ist, erzeugt die Ultraschall-Sendeeinrichtung 39 ein Ultraschall-Sendesignal mit einer Anfangsamplitude $A_0$ und koppelt dies in die Messstrecke 37 ein.

Wie schematisch in **Fig. 2** dargestellt, durchläuft das Ultraschall-Sendesignal 42 zunächst das Referenzmaterial 38, bis es die Grenzfläche 43 zwischen Referenzmaterial 38 und Prüfmaterial 02 erreicht. Ein Teil des Ultraschall-Sendesignals durchtritt die Grenzfläche 43 und durchläuft das Prüfmaterial 02, bis es die Ultraschall-Empfangseinrichtung 41 als transmittiertes Ultraschall-Empfangssignal 44 erreicht. Durch Auswertung des transmittierten Ultraschall-Empfangssignal 44 kann anschließend die Durchlaufzeit $t_G$, die das transmittierte Ultraschall-Empfangssignal zum Durchlaufen der gesamten Messstrecke 37 benötigt hat, und die Amplitude $A_T$ des transmittierten Ultraschall-Empfangssignals 44 gemessen bzw. ermittelt werden.

Der an der Grenzfläche 43 reflektierte Anteil des Ultraschall-Empfangssignals 44 durchläuft das Referenzmaterial 38 in Gegenrichtung ein zweites Mal, bis er die durch Umschalten der Ultraschall-Sendeeinrichtung 39 gebildete Ultraschall-Empfangseinrichtung 40 als reflektiertes Ultraschall-Empfangssignal 45 erreicht. Durch Auswertung der Messwerte der Ultraschall-Empfangseinrichtung 40 kann die Amplitude $A_R$ des reflektierten Ultraschall-Empfangssignals 45 ermittelt werden. Die Art der Weiterverarbeitung der Messsignale der beiden Ultraschall-Empfangseinrichtungen 41 wird nachfolgend anhand der Skizze in Fig. 1 weiter erläutert.

Die Vorrichtung 01 umfasst neben der kombinierten Ultraschall-Sende- und Empfangseinrichtung 39/40 und der Ultraschall-Empfangseinrichtung 41 ein Signalvorbereitungsmodul 05 zur Generierung der an der Ultraschall-Sendeeinrichtung 39 abzustrahlenden Ultraschallsignale. Weiter ist ein Signalgenerator 06 und Signalverstärker 07 vorgesehen.

Die von den Ultraschall-Empfangseinrichtungen 40 bzw. 41 aufgezeichneten Signalverläufe werden zunächst mittels Signalverstärkern 08 verstärkt. Die transmittierten Ultraschall-Empfangssignale 44 werden anschließend in einer Signalweiche 09 aufgeteilt und zur parallelen Verarbeitung auf eine erste Verarbeitungsreinrichtung 10 und eine zweite Verarbeitungseinrichtung 11 verteilt. Die erste Verarbeitungseinrichtung 10 dient der Bestimmung der Durchlaufzeit, die das transmittierte Ultraschall-Empfangssignal 44 bei der jeweils eingestellten Frequenz zum Durchlauf der Messstrecke 37 benötigt hat. Nach Durchlauf eines Signalaufbereitungsmoduls 12 gelangt das transmittierte Ultraschall-Empfangssignal 44 in einen Time-to-digital-Converter 13, mit dem die Durchlaufzeit des Ultraschallsignals, d.h. die Zeit zwischen der Abstrahlung an der Ultraschall-Sendeeinrichtung 39 bis zum Empfang des transmittierten Ultraschall-Empfangssignals 44 an der Ultraschall-Empfangseinrichtung 41 gemessen werden kann.

Die Funktion des Time-to-digital-Converters 13 beruht dabei darauf, dass die Durchlaufzeit eines elektrischen Signals durch eine Vielzahl von in dem Time-to-digital-Converter 13 enthaltenen elektronischen Schaltkreisen 14 bekannt ist.

Zur Zeitmessung der Durchlaufzeit wird das Auslösesignal des Ultraschall-Sendesignals zeitgleich mit der Abstrahlung an der Ultraschall-Sendeeinrichtung 39 auch auf den Time-to-digital-Converter 13 geleitet, um den Zeitmessprozess zu starten. Das Auslösesignal des Ultraschall-Sendesignals durchläuft dann die hintereinander angeordneten Schaltkreise 14 im TTD-Converter 13. Jeder Durchlauf eines elektronischen Schaltkreises 14, der einer vorbestimmten Durchlaufzeit entspricht, wird dabei von einem Zähler 15 addiert. Sobald dann das Ultraschall-Empfangssignal 44 auf die elektronischen Schaltkreise 14 geleitet wird, schaltet der Zähler 15 ab und multipliziert die Anzahl der addierten Durchläufe mit der bekannten Durchlaufzeit der einzelnen elektronischen Schaltkreise 14. Daraus ergibt sich dann insgesamt die Durchlaufzeit $t_D$ für die entsprechende Messung. Für jede Sendefrequenz ergibt sich eine Durchlaufzeit ($t_G$) 16, die jeweils zugeordnet zu der jeweiligen Sendefrequenz in einer Speichereinrichtung 17 abgespeichert wird.

Parallel dazu wird in der zweiten Verarbeitungseinrichtung 11 das transmittierte Ultraschall-Empfangssignal 44 ausgewertet und die Amplitude $A_T$ des transmittierten Ultraschall-Empfangssignals 20 ermittelt. Dazu ist in der zweiten Verarbeitungseinrichtung 11 ein Signalaufbereitungsmodul 18 vorgesehen, mit dem das transmittierte Ultraschall-Empfangssignal 44 aufbereitet und die Amplitude ($A_T$) 20 des Ultraschall-Empfangssignals 44 ermittelt wird. Mit der Signalweiche 19 wird das Ultraschall-Empfangssignal 44 aufgesplittet und indexiert. In einer dritten Verarbeitungseinrichtung 46 wird das reflektierte Ultraschall-Empfangssignal 45 mittels eines Signalaufbereitungsmoduls 18 aufbereitet und die Amplitude ($A_R$) 47 des reflektierten Ultraschall-Empfangssignals ermittelt. Die Ermittlung der Amplitudenwerte 20 und 47 in den Verarbeitungseinrichtungen 11 und 46 erfolgt dabei jeweils frequenzabhängig und die Ergebnisdaten werden zur jeweiligen Frequenz in der Speichereinrichtung 17 zusammen mit der jeweiligen Durchlaufzeit 16 abgespeichert.

Die in der Speichereinrichtung 17 abgespeicherten Ergebnisdaten, nämlich die Durchlaufzeit ($t_G$) 16, die Amplitude ($A_T$) 20 des transmittierten Ultraschall-Empfangssignals 44 und die Amplitude ($A_R$) 47 des reflektierten Ultraschall-Empfangssignals 45, werden anschließend aus der Speichereinrichtung 17 ausgelesen und an eine Auswerteeinrichtung 48 zur Weiterberechnung der Ergebnisdaten weitergeleitet.

**Fig. 3** stellt die Formelsammlung zur Berechnung akustischer Materialkennwerte, wie sie die Auswerteeinrichtung 48 bei der Berechnung der Ergebnisdaten voraussetzt, dar.

**Fig. 4** zeigt schematisch die Auswerteeinrichtung 48, mit der mit Hilfe der in Fig. 4 dargestellten Formeln die Ergebnisdaten für die akustische Impedanz $Z_m$ des Prüfmaterials, der Reflektionsgrad R an der Grenzfläche 43, der akustische Dämpfungskoeffizient $\alpha_M$ des Prüfmaterials, die Schallübertragungsgeschwindigkeit $c_M$ des Prüfmaterials, die Dichte $\rho_M$ des Prüfmaterials und die Kompressibilität des Prüfmaterials aus den Messwerten, nämlich der Durchlaufzeit $t_G$, der Amplitude ($A_T$) des transmittierten Ultraschall-Empfangssignals 24 und/oder der Amplitude ($A_R$) des reflektierten Ultraschall-Empfangssignals 45 berechnet werden können.

Nach der Bestimmung der akustischen Materialkennwerte des Transformatorenöls 30 durch Berechnung in der Auswerteeinrichtung 48 kann in einem nächsten Schritt der Wassergehalt bzw. der Säuregehalt im Transformatorenöl 30 bestimmt werden. Zusätzlich können auch weitere Isolationsparameter wie die Durchschlagsspannung (nach BDV) sowie der Verlustfaktor (dissipation factor: Tangens Delta) bestimmt werden.

**Patentansprüche**

1. Vorrichtung (01) zur Untersuchung eines Prüfmaterials (02) durch akustische Spektroskopie, mit einer Messstrecke (37), die aus einem Referenzmaterial (38) und dem Prüfmaterial (02) gebildet ist, wobei die akustischen Materialkennwerte des Referenzmaterials (38) für unterschiedliche Frequenzen, nämlich die Dichte ($\rho_R$), die Schallübertragungsgeschwindigkeit ($c_R$), der akustische Dämpfungskoeffizient ($\alpha_R$) und die akustische Impedanz ($Z_R$), bekannt sind, und wobei die Länge ($x_R$) des Referenzmaterials (38) bekannt ist, und wobei die Länge ($x_M$) des Prüfmaterials (02) bekannt ist, und mit einer Ultraschall-Sendeeinrichtung (39) zum Aussenden eines Ultraschall-Sendesignals (42) mit einer Anfangsamplitude ($A_0$) durch die Messstrecke (37), und mit einer ersten Ultraschall-Empfangseinrichtung (41) zum Empfangen des transmittierten Ultraschall-Empfangssignals (44) nach Durchlaufen der Messstrecke (37), und mit einer zweiten Ultraschall-Empfangseinrichtung (40) zum Empfangen des an der Grenzfläche (43) zwischen Prüfmaterial (02) und Referenzmaterials (38) reflektierten Ultraschall-Empfangssignals (45) nach zweimaligem Durchlaufen des Referenzmaterials (38) oder Prüfmaterials, wobei die Sendeeinrichtung (39) zum Geben von Ultraschall-Sendesignalen (42) unterschiedlicher Frequenz (f) und die beiden Empfangseinrichtungen (40, 41) zum Empfangen entsprechender Ultraschall-Empfangssignale (44, 45) unterschiedlicher Frequenz ausgebildet sind,

   wobei mit einer ersten Verarbeitungseinrichtung (10) der Vorrichtung (01) für die Ultraschall-Sendesignale (42) die Durchlaufzeit ($t_G$) der zugeordneten, transmittierten Ultraschall-Empfangssignale (44) nach Durchlaufen der Messstrecke (37) gemessen werden kann, mit einer zweiten Verarbeitungseinrichtung (11) der Vorrichtung (01) für die Ultraschall-Sendesignale (42) die Amplitude ($A_T$) der zugeordneten, transmittierten Ultraschall-Empfangssignale (44) nach Durchlaufen der Messstrecke (37) ermittelt werden kann,
   und mit einer dritten Verarbeitungseinrichtung (46) der Vorrichtung (01) für die Ultraschall-Sendesignale (42) die Amplitude ($A_R$) der zugeordneten, an der Grenzfläche (43) zwischen Prüfmaterial (02) und Referenzmaterial (38) reflektierten Ultraschall-Empfangssignale (45) nach zweimaligem Durchlaufen des Referenzmaterials (38) oder Prüfmaterials ermittelt werden kann,
   und wobei mit einer Auswerteeinrichtung (48) der Vorrichtung (01) für unterschiedliche Frequenzen (f) die Dichte ($\rho_M$) durch die Formel

   $$\frac{Z_R(t_G \cdot c_R - x_M - x_R) \cdot (A_0 - A_R \cdot e^{2\alpha_R \cdot x_R})}{x_M \cdot c_R \cdot (A_0 + A_R \cdot e^{2\alpha_R \cdot x_R})},$$

   die Schallübertragungsgeschwindigkeit ($c_M$) durch die Formel

   $$\frac{x_M \cdot c_R}{t_G \cdot c_R - x_M - x_R},$$

   der akustische Dämpfungskoeffizient ($\alpha_M$) durch die Formel

   $$-\frac{1}{x_M} \cdot \ln \frac{A_T \cdot e^{2\alpha_R \cdot x_R}}{A_0 - A_R \cdot e^{2\alpha_R \cdot x_R}}$$

   und/oder
   die akustische Impedanz ($Z_M$) durch die Formel

   $$Z_R \cdot \frac{A_0 - A_R \cdot e^{2\alpha_R \cdot x_R}}{A_0 + A_R \cdot e^{2\alpha_R \cdot x_R}}$$

   berechnet werden kann.

2. Vorrichtung nach Anspruch 1,
   wobei in der ersten Verarbeitungseinrichtung (10) die Durchlaufzeit ($t_G$) mit einer Auflösung von zumindest 100 Pikosekunden, insbesondere mit einer Auflösung von 10 Pikosekunden, ermittelt werden kann.

3. Vorrichtung nach Anspruch 1 oder 2,
   wobei die erste Verarbeitungseinrichtung (10) einen Time-to-digital-Converter umfasst, mit dem die Durchlaufzeit ($t_G$)

ermittelt wird.

4. Vorrichtung nach einem der Ansprüche 1 bis 3, wobei die zweite Ultraschall-Empfangseinrichtung (40) zum Empfangen des an der Grenzfläche (43) zwischen Prüfmaterial (02) und Referenzmaterial (38) reflektierten Ultraschall-Empfangssignals (45) nach zweimaligem Durchlaufen des Referenzmaterials (38) oder Prüfmaterials durch Umschalten der Ultraschall-Sendeeinrichtung (39) vom Sendemodus in den Empfangsmodus realisiert wird.

5. Vorrichtung nach einem der Ansprüche 1 bis 4, wobei das Referenzmaterial (38) aus einem Festkörper, insbesondere aus Kunststoff oder Glas, besteht.

6. Vorrichtung nach einem der Ansprüche 1 bis 5, wobei zwischen der Grenzfläche (43) des Referenzmaterials (38) und der ersten Ultraschall-Empfangseinrichtung (41) ein Hohlraum (35) vorhanden ist, der bei der Untersuchung mit einem flüssigen oder gasförmigen Prüfmaterial (02), insbesondere mit Transformatorenöl (30), befüllt oder durchströmt werden kann.

7. Transformator (31) zur Wandlung einer Eingangswechselspannung in eine Ausgangswechselspannung, wobei der Transformator ein mit Transformatorenöl (30) gefülltes Gehäuse (36) umfasst, und in oder am Transformator (31) eine Vorrichtung (01) nach einem der vorhergehenden Ansprüche zur Online-Untersuchung des Transformatorenöls (30) vorgesehen ist.

8. Verfahren zur Untersuchung eines biologischen oder nichtbiologischen Prüfmaterials (02) durch akustische Spektroskopie,

mit einer Messstrecke (37), die aus einem Referenzmaterial (38) und dem Prüfmaterial (02) gebildet ist, wobei die akustischen Materialkennwerte des Referenzmaterials (38) für unterschiedliche Frequenzen, nämlich die Dichte ($\rho_R$), die Schallübertragungsgeschwindigkeit ($c_R$), der akustische Dämpfungskoeffizient ($\alpha_R$) und die akustische Impedanz ($Z_R$), bekannt sind, und wobei die Länge ($x_R$) des Referenzmaterials (38) bekannt ist, und wobei die Länge ($x_M$) des Prüfmaterials (02) bekannt ist, und mit einer Ultraschall-Sendeeinrichtung (39) zum Aussenden eines Ultraschall-Sendesignals (42) mit einer Anfangsamplitude ($A_0$) durch die Messstrecke (37), und mit einer ersten Ultraschall-Empfangseinrichtung (41) zum Empfangen des transmittierten Ultraschall-Empfangssignals (44) nach Durchlaufen der Messstrecke (37), und mit einer zweiten Ultraschall-Empfangseinrichtung (40) zum Empfangen des an der Grenzfläche (43) zwischen Prüfmaterial (02) und Referenzmaterial (38) reflektierten Ultraschall-Empfangssignals (45) nach zweimaligem Durchlaufen des Referenzmaterials (38) oder Prüfmaterials, wobei die Sendeeinrichtung (39) zum Geben von Ultraschall-Sendesignalen (42) unterschiedlicher Frequenz (f) und die beiden Empfangseinrichtungen (40, 41) zum Empfangen entsprechender Ultraschall-Empfangssignale (44, 45) unterschiedlicher Frequenz (f) ausgebildet sind,
wobei mehrere Ultraschall-Sendesignale (42) unterschiedlicher Frequenz (f) in die Messstrecke (37) ausgesendet werden, wobei

a) für die unterschiedlichen Ultraschall-Sendesignale (42) die Durchlaufzeit ($t_G$) der zugeordneten, transmittierten Ultraschall-Empfangssignale (44) nach Durchlaufen der Messstrecke (37) gemessen wird,
b) für die unterschiedlichen Ultraschall-Sendesignale (42) die Amplitude ($A_T$) der zugeordneten, transmittierten Ultraschall-Empfangssignale (44) nach Durchlaufen der Messstrecke (37) ermittelt wird,
c) für die unterschiedlichen Ultraschall-Sendesignale (42) die Amplitude ($A_R$) der zugeordneten, an der Grenzfläche (43) zwischen Prüfmaterial (02) und Referenzmaterial (38) reflektierten Ultraschall-Empfangssignale (45) nach zweimaligem Durchlaufen des Referenzmaterials (38) oder Prüfmaterials ermittelt wird,
d) die Dichte ($\rho_M$) durch die Formel

$$\frac{Z_R(t_G \cdot c_R - x_M - x_R) \cdot (A_0 - A_R \cdot e^{2\alpha_R \cdot x_R})}{x_M \cdot c_R \cdot (A_0 + A_R \cdot e^{2\alpha_R \cdot x_R})},$$

die Schallübertragungsgeschwindigkeit ($c_M$) durch die Formel

$$\frac{x_M \cdot c_R}{t_G \cdot c_R - x_M - x_R},$$

der akustische Dämpfungskoeffizient ($\alpha_M$) durch die Formel

$$-\frac{1}{x_M}\cdot\ln\frac{A_T\cdot e^{2\alpha_R\cdot x_R}}{A_0-A_R\cdot e^{2\alpha_R\cdot x_R}}$$

und/oder die akustische Impedanz ($Z_M$) durch die Formel

$$Z_R\cdot\frac{A_0-A_R\cdot e^{2\alpha_R\cdot x_R}}{A_0+A_R\cdot e^{2\alpha_R\cdot x_R}}$$

für unterschiedliche Frequenzen berechnet wird.

**9.** Verfahren nach Anspruch 8,
wobei die Durchlaufzeit ($t_G$) mit einer Auflösung von zumindest 100 Pikosekunden, insbesondere mit einer Auflösung von 10 Pikosekunden, ermittelt wird.

**10.** Verfahren nach Anspruch 8 oder 9,
wobei die akustischen Materialkennwerte eines Öls, insbesondere eines Transformatorenöls (30), insbesondere die Dichte ($\rho_M$) des Transformatorenöls (30), die Schallübertragungsgeschwindigkeit ($c_M$) im Transformatorenöl (30), der akustische Dämpfungskoeffizient ($\alpha_M$) des Transformatorenöls (30) und/oder die akustische Impedanz ($Z_M$) des Transformatorenöls (30), ermittelt werden.

**11.** Verfahren nach Anspruch 10,
wobei aus den akustischen Materialkennwerten des Transformatorenöls (30), insbesondere aus der Dichte ($\rho_M$) des Transformatorenöls (30), der Schallübertragungsgeschwindigkeit ($c_M$) im Transformatorenöl (30), dem akustischen Dämpfungskoeffizienten ($\alpha_M$) des Transformatorenöls (30) und/oder der akustischen Impedanz ($Z_M$) des Transformatorenöls (30), der Wassergehalt und/oder der Säuregehalt im Transformatorenöl (30) abgeleitet wird.

**12.** Verfahren nach einem der Ansprüche 8 bis 11,
wobei die Ultraschall-Sendesignale (42) unterschiedlicher Frequenz (f) in der Ultraschall-Sendeeinrichtung (39) auf ein gemeinsames Trägersignal aufmoduliert oder aufaddiert und danach gemeinsam ausgesendet werden.

## Claims

**1.** A device (01) for examining a test material (02) via acoustic spectroscopy, comprising a measuring distance (37) which is formed from a reference material (38) and the test material (02), the acoustic material parameters of the reference material (38), i.e., the density ($\rho_R$), the sonic transmission speed ($c_R$), the acoustic attenuation coefficient ($\alpha_R$) and the acoustic impedance ($Z_R$), being known for different frequencies, and the length ($x_R$) of the reference material (38) being known, and the length ($x_M$) of the test material (02) being known, and comprising an ultrasonic transmission device (39) for transmitting an ultrasonic transmission signal (42) having an initial amplitude ($A_0$) through the measuring distance (37), and comprising a first ultrasonic reception device (41) for receiving the transmitted ultrasonic reception signal (44) after said signal (44) has passed through the measuring distance (37),

and comprising a second ultrasonic reception device (40) for receiving the ultrasonic reception signal (45) reflected on the boundary surface (43) between the test material (02) and the reference material (38) after said signal (45) has twice passed through the reference material (38) or test material, said transmission device (39) being configured for giving ultrasonic transmission signals (42) having different frequencies (f) and the two reception devices (40, 41) being configured for receiving corresponding ultrasonic reception signals (44, 45) having different frequencies,
wherein the time of flight ($t_G$) of the allocated transmitted ultrasonic reception signals (44) can be measured, after said signals (44) have passed through the measuring distance (37), using a first processing device (10) of the device (01) for the ultrasonic transmission signals (42),
and wherein the amplitude ($A_T$) of the allocated transmitted ultrasonic reception signals (44) can be identified, after said signals (44) have passed through the measuring distance (37), using a second processing device (11) of the device (01) for the ultrasonic transmission signals (42),

and wherein the amplitude ($A_R$) of the allocated ultrasonic reception signals (45) reflected on the boundary surface (43) between the test material (02) and the reference material (38) can be identified, after said signals (45) have twice passed through the reference material (38) or test material, using a third processing device (46) of the device (01) for the ultrasonic transmission signals (42), and wherein the density ($\rho_M$) can be calculated by the formula

$$\frac{Z_R(t_G \cdot c_R - x_M - x_R) \cdot (A_0 - A_R \cdot e^{2\alpha_R \cdot x_R})}{x_M \cdot c_R \cdot (A_0 + A_R \cdot e^{2\alpha_R \cdot x_R})},$$

the sonic transmission speed ($c_M$) can be calculated by the formula

$$\frac{x_M \cdot c_R}{t_G \cdot c_R - x_M - x_R},$$

the acoustic attenuation coefficient ($\alpha_M$) can be calculated by the formula

$$-\frac{1}{x_M} \cdot \ln \frac{A_T \cdot e^{2\alpha_R \cdot x_R}}{A_0 - A_R \cdot e^{2\alpha_R \cdot x_R}},$$

and/or the acoustic impedance ($Z_M$) can be calculated by the formula

$$Z_R \cdot \frac{A_0 - A_R \cdot e^{2\alpha_R \cdot x_R}}{A_0 + A_R \cdot e^{2\alpha_R \cdot x_R}}$$

for different frequencies (f) using an evaluation device (48) of the device (01).

**2.** The device according to claim 1, wherein the time of flight ($t_G$) can be identified with a resolution of at least 100 picoseconds, in particular with a resolution of 10 picoseconds, in the first processing device (10).

**3.** The device according to claim 1 or 2, wherein the first processing device (10) comprises a time-to-digital converter by means of which the time of flight ($t_G$) is identified.

**4.** The device according to any one of the claims 1 to 3, wherein the second ultrasonic reception device (40) is configured for receiving the ultrasonic reception signal (45) reflected on the boundary surface (43) between the test material (02) and the reference material (38), after said signal (45) has twice passed through the reference material (38) or the test material, via switching the ultrasonic transmission device (39) from the transmission mode to the reception mode.

**5.** The device according to any one of the claims 1 to 4, wherein the reference material (38) consists of a solid body, in particular of plastic or glass.

**6.** The device according to any one of the claims 1 to 5, wherein a cavity (35) exists between the boundary surface (43) of the reference material (38) and the first ultrasonic reception device (41), a liquid or gaseous test material (02), in particular transformer oil (30), being able to fill or flow through said cavity (35) during examination.

**7.** A transformer (31) for converting an AC input voltage to an AC output voltage, wherein said transformer comprises a housing (36) filled with transformer oil (30), and wherein a device (01) according to any one of the preceding claims is provided in or on the transformer (31) for the online examination of the transformer oil (30).

**8.** A method for examining a biological or non-biological test material (02) via acoustic spectroscopy, comprising a measuring distance (37) which is formed from a reference material (38) and the test material (02), the acoustic material parameters of the reference material (38), i.e., the density ($\rho_R$), the sonic transmission speed ($c_R$), the acoustic attenuation coefficient ($\alpha_R$) and the acoustic impedance ($Z_R$), being known for different frequencies, and the length ($x_R$) of the reference material (38) being known, and the length ($x_M$) of the test material (02) being known, and comprising an ultrasonic transmission device (39) for transmitting an ultrasonic transmission signal (42) having an initial amplitude ($A_0$) through the measuring distance (37), and comprising a first ultrasonic reception device (41) for receiving the transmitted ultrasonic reception signal (44) after said signal (44) has passed through the measuring distance (37), and comprising a second ultrasonic reception device (40) for receiving the ultrasonic reception signal (45) reflected on the boundary surface (43) between the test material (02) and the reference material (38) after said signal (45) has twice passed through the reference material (38) or test material, said transmission device (39) being configured for giving ultrasonic transmission signals (42) having different frequencies (f) and the two reception devices (40, 41) being configured for receiving corresponding ultrasonic reception signals (44, 45) having different frequencies (f),
wherein several ultrasonic transmission signals (42) having different frequencies (f) are emitted into the measuring distance (37), wherein

a) the time of flight ($t_G$) of the allocated transmitted ultrasonic reception signals (44) is measured for the different ultrasonic transmission signals (42) after said signals (44) have passed through the measuring distance (37),
b) the amplitude ($A_T$) of the allocated transmitted ultrasonic reception signals (44) is identified for the different ultrasonic transmission signals (42) after said signals (44) have passed through the measuring distance (37),
c) the amplitude ($A_R$) of the allocated ultrasonic reception signals (45) reflected on the boundary surface (43) between the test material (02) and the reference material (38) is identified for the different ultrasonic transmission signals (42) after said signals (45) have twice passed through the reference material (38) or test material,
d) the density ($\rho_M$), is calculated by the formula

$$\frac{Z_R(t_G \cdot c_R - x_M - x_R) \cdot (A_0 - A_R \cdot e^{2\alpha_R x_R})}{x_M \cdot c_R \cdot (A_0 + A_R \cdot e^{2\alpha_R x_R})},$$

the sonic transmission speed ($c_M$) is calculated by the formula

$$\frac{x_M \cdot c_R}{t_G \cdot c_R - x_M - x_R},$$

the acoustic attenuation coefficient ($\alpha_M$) is calculated by the formula

$$-\frac{1}{x_M} \cdot \ln \frac{A_T \cdot e^{2\alpha_R \cdot x_R}}{A_0 - A_R \cdot e^{2\alpha_R \cdot x_R}},$$

and/or the acoustic impedance ($Z_M$) is calculated by the formula

$$Z_R \cdot \frac{A_0 - A_R \cdot e^{2\alpha_R \cdot x_R}}{A_0 + A_R \cdot e^{2\alpha_R \cdot x_R}}$$

for different frequencies.

**9.** The method according to claim 8, wherein the time of flight ($t_G$) is identified with a resolution of at least 100 picoseconds, in particular with a resolution of 10 picoseconds.

**10.** The method according to claim 8 or 9,
wherein the acoustic material parameters of an oil, in particular a transformer oil (30), in particular the density ($\rho_M$) of the transformer oil (30), the sonic transmission speed ($c_M$) in the transformer oil (30), the acoustic attenuation coefficient ($\alpha_M$) of the transformer oil (30) and/or the acoustic impedance ($Z_M$) of the transformer oil (30), are identified.

**11.** The method according to claim 10,
wherein the water content and/or the acidity level in the transformer oil (30) is derived from the acoustic material parameters of the transformer oil (30), in particular from the density ($\rho_M$) of the transformer oil (30), the sonic transmission speed ($c_M$) in the transformer oil (30), the acoustic attenuation coefficient ($\alpha_M$) of the transformer oil (30) and/or the acoustic impedance ($Z_M$) of the transformer oil (30).

**12.** The method according to any one of the claims 8 to 11,
wherein the ultrasonic transmission signals (42) having different frequencies (f) are modulated onto or added to a shared carrier signal and are subsequently transmitted together in the ultrasonic transmission device (39).

## Revendications

**1.** Dispositif (01) pour examiner un matériau d'essai (02) par spectroscopie acoustique, comprenant une distance de mesure (37) formée d'un matériau de référence (38) et du matériau d'essai (02), les paramètres de matériau acoustiques du matériau de référence (38), c'est-à-dire la densité ($\rho_R$), la vitesse de transmission sonore ($c_R$), le coefficient d'atténuation ($\alpha_R$) acoustique et l'impédance ($Z_R$) acoustique, étant connus pour différentes fréquences, et la longueur ($x_R$) du matériau de référence (38) étant connue, et la longueur ($x_M$) du matériau d'essai (02) étant connue, et comprenant un dispositif de transmission (39) ultrasonique pour transmettre un signal de transmission (42) ultrasonique ayant une amplitude initiale ($A_0$) à travers la distance de mesure (37), et comprenant un premier dispositif de réception (41) ultrasonique pour recevoir le signal de réception (44) ultrasonique transmis après que ledit signal (44) a traversé la distance de mesure (37),

et comprenant un deuxième dispositif de réception (40) ultrasonique pour recevoir le signal de réception (45) ultrasonique réfléchi sur la surface de séparation (43) entre le matériau d'essai (02) et le matériau de référence (38) après que ledit signal (45) a traversé deux fois le matériau de référence (38) ou le matériau d'essai, ledit dispositif de transmission (39) étant configuré pour émettre des signaux de transmission (42) ultrasoniques ayant des différentes fréquences (f) et les deux dispositifs de réception (40, 41) étant configurés pour recevoir des signaux de réception (44, 45) ultrasoniques correspondants ayant des différentes fréquences, dans lequel le temps de vol ($t_G$) des signaux de réception (44) ultrasoniques transmis attribués peut être mesuré, après que lesdits signaux (44) ont traversé la distance de mesure (37), à l'aide d'un premier dispositif de traitement (10) du dispositif (01) pour les signaux de transmission (42) ultrasoniques,
et dans lequel l'amplitude ($A_T$) des signaux de réception (44) ultrasoniques transmis attribués peut être identifiée, après que lesdits signaux (44) ont traversé la distance de mesure (37), à l'aide d'un deuxième dispositif de traitement (11) du dispositif (01) pour les signaux de transmission (42) ultrasoniques,
et dans lequel l'amplitude ($A_R$) des signaux de réception (45) ultrasoniques attribués réfléchis sur la surface de séparation (43) entre le matériau d'essai (02) et le matériau de référence (38), peut être identifiée, après que lesdits signaux (45) ont traversé deux fois le matériau de référence (38) ou le matériau d'essai, à l'aide d'un troisième dispositif de traitement (46) du dispositif (01) pour les signaux de transmission (42) ultrasoniques,
et dans lequel la densité ($\rho_M$) peut être calculée par la formule

$$\frac{Z_R(t_G \cdot c_R - x_M - x_R) \cdot (A_0 - A_R \cdot e^{2\alpha_R \cdot x_R})}{x_M \cdot c_R \cdot (A_0 + A_R \cdot e^{2\alpha_R \cdot x_R})},$$

la vitesse de transmission ($c_M$) sonore peut être calculée par la formule

$$\frac{x_M \cdot c_R}{t_G \cdot c_R - x_M - x_R},$$

le coefficient d'atténuation ($\alpha_M$) acoustique peut être calculé par la formule

$$-\frac{1}{x_M} \cdot \ln \frac{A_T \cdot e^{2\alpha_R \cdot x_R}}{A_0 - A_R \cdot e^{2\alpha_R \cdot x_R}},$$

et/ou l'impédance ($Z_M$) acoustique peut être calculée par la formule

$$Z_R \cdot \frac{A_0 - A_R \cdot e^{2\alpha_R \cdot x_R}}{A_0 + A_R \cdot e^{2\alpha_R \cdot x_R}}$$

pour différentes fréquences (f) à l'aide d'un dispositif d'évaluation (48) du dispositif (01).

**2.** Dispositif selon la revendication 1,
dans lequel le temps de vol ($t_G$) peut être identifié avec une résolution d'au moins 100 picosecondes, en particulier avec une résolution de 10 picosecondes, dans le premier dispositif de traitement (10).

**3.** Dispositif selon la revendication 1 ou la revendication 2,
dans lequel le premier dispositif de traitement (10) comprend un convertisseur temps-numérique au moyen duquel le temps de vol ($t_G$) est identifié.

**4.** Dispositif selon l'une quelconque des revendications 1 à 3, dans lequel le deuxième dispositif de réception (40) ultrasonique est configuré pour recevoir le signal de réception (45) ultrasonique réfléchi sur la surface de séparation (43) entre le matériau d'essai (02) et le matériau de référence (38), après que ledit signal (45) a traversé deux fois le matériau de référence (38) ou le matériau d'essai, en faisant passer le dispositif de transmission (39) ultrasonique du mode de transmission au mode de réception.

**5.** Dispositif selon l'une quelconque des revendications 1 à 4, dans lequel le matériau de référence (38) consiste en un corps solide, notamment en plastique ou en verre.

**6.** Dispositif selon l'une quelconque des revendications 1 à 5, dans lequel une cavité (35) existe entre la surface de séparation (43) du matériau de référence (38) et le premier dispositif de réception (41) ultrasonique, un matériau d'essai (02) liquide ou gazeux, notamment de l'huile de transformateur (30), pouvant remplir ou s'écouler à travers ladite cavité (35) pendant l'examen.

**7.** Transformateur (31) pour convertir une tension d'entrée CA en une tension de sortie CA, dans lequel ledit transformateur comprend un boîtier (36) rempli d'huile de transformateur (30),
et dans lequel un dispositif (01) selon l'une quelconque des revendications précédentes est prévu dans ou sur le transformateur (31) pour l'examen en ligne de l'huile de transformateur (30).

**8.** Procédé d'examen d'un matériau d'essai (02) biologique ou non biologique par spectroscopie acoustique, comprenant une distance de mesure (37) formée d'un matériau de référence (38) et du matériau d'essai (02), les paramètres de matériau acoustiques du matériau de référence (38), c'est-à-dire la densité ($\rho_R$), la vitesse de transmission sonore ($c_R$), le coefficient d'atténuation ($\alpha_R$) acoustique et l'impédance ($Z_R$) acoustique, étant connus pour différentes fréquences, et la longueur ($x_R$) du matériau de référence (38) étant connue, et la longueur ($x_M$) du matériau d'essai (02) étant connue, et comprenant un dispositif de transmission (39) ultrasonique pour transmettre un signal de transmission (42) ultrasonique ayant une amplitude initiale ($A_0$) à travers la distance de mesure (37), et comprenant un premier dispositif de réception (41) ultrasonique pour recevoir le signal de réception (44) ultrasonique transmis après que ledit signal (44) a traversé la distance de mesure (37),

et comprenant un deuxième dispositif de réception (40) ultrasonique pour recevoir le signal de réception (45) ultrasonique réfléchi sur la surface de séparation (43) entre le matériau d'essai (02) et le matériau de référence (38) après que ledit signal (45) a traversé deux fois le matériau de référence (38) ou le matériau d'essai, ledit dispositif de transmission (39) étant configuré pour émettre des signaux de transmission (42) ultrasoniques ayant des différentes fréquences (f) et les deux dispositifs de réception (40, 41) étant configurés pour recevoir des signaux de réception (44, 45) ultrasoniques correspondants ayant des différentes fréquences,
dans lequel plusieurs signaux de transmission (42) ultrasoniques ayant différentes fréquences (f) sont émis dans la distance de mesure (37), dans lequel

a) le temps de vol ($t_G$) des signaux de réception (44) ultrasoniques transmis attribués est mesuré pour les différents signaux de transmission (42) ultrasoniques après que lesdits signaux (44) ont traversé la distance de mesure (37),
b) l'amplitude ($A_T$) des signaux de réception (44) ultrasoniques transmis attribués est identifiée pour les différents signaux de transmission (42) ultrasoniques après que lesdits signaux (44) ont traversé la distance de mesure (37),

c) l'amplitude ($A_R$) des signaux de réception (45) ultrasoniques attribués réfléchis sur la surface de séparation (43) entre le matériau d'essai (02) et le matériau de référence (38) est identifiée pour les différents signaux de transmission (42) ultrasoniques après que lesdits signaux (45) ont traversé deux fois le matériau de référence (38) ou le matériau d'essai,
d) la densité ($\rho_M$) est calculée par la formule suivante

$$\frac{Z_R(t_G \cdot c_R - x_M - x_R) \cdot (A_0 - A_R \cdot e^{2\alpha_R \cdot x_R})}{x_M \cdot c_R \cdot (A_0 + A_R \cdot e^{2\alpha_R \cdot x_R})},$$

la vitesse de transmission ($c_M$) sonore est calculée par la formule

$$\frac{x_M \cdot c_R}{t_G \cdot c_R - x_M - x_R},$$

le coefficient d'atténuation ($\alpha_M$) acoustique est calculé par la formule

$$-\frac{1}{x_M} \cdot \ln \frac{A_T \cdot e^{2\alpha_R \cdot x_R}}{A_0 - A_R \cdot e^{2\alpha_R \cdot x_R}},$$

et/ou l'impédance ($Z_M$) acoustique est calculée par la formule

$$Z_R \cdot \frac{A_0 - A_R \cdot e^{2\alpha_R \cdot x_R}}{A_0 + A_R \cdot e^{2\alpha_R \cdot x_R}}$$

pour différentes fréquences.

**9.** Procédé selon la revendication 8,
dans lequel le temps de vol ($t_G$) est identifié avec une résolution d'au moins 100 picosecondes, en particulier avec une résolution de 10 picosecondes.

**10.** Procédé selon la revendication 8 ou la revendication 9,
dans lequel les paramètres de matériau acoustiques d'une huile, notamment d'une huile de transformateur (30), notamment la densité ($\rho_M$) de l'huile de transformateur (30), la vitesse de transmission ($c_M$) sonore dans l'huile de transformateur (30), le coefficient d'atténuation ($\alpha_M$) acoustique de l'huile de transformateur (30) et/ou l'impédance ($Z_M$) acoustique de l'huile de transformateur (30), sont identifiés.

**11.** Procédé selon la revendication 10,
dans lequel la teneur en eau et/ou le niveau d'acidité dans l'huile de transformateur (30) est dérivé/e des paramètres de matériau acoustiques de l'huile de transformateur (30), notamment de la densité ($\rho_M$) de l'huile de transformateur (30), la vitesse de transmission ($c_M$) sonore dans l'huile de transformateur (30), le coefficient d'atténuation ($\alpha_M$) acoustique de l'huile de transformateur (30) et/ou l'impédance ($Z_M$) acoustique de l'huile de transformateur (30).

**12.** Procédé selon l'une quelconque des revendications 8 à 11,
dans lequel les signaux de transmission (42) ultrasoniques ayant différentes fréquences (f) sont modulés sur ou ajoutés à un signal porteur partagé et sont ensuite transmis ensemble dans le dispositif de transmission (39) ultrasonique.

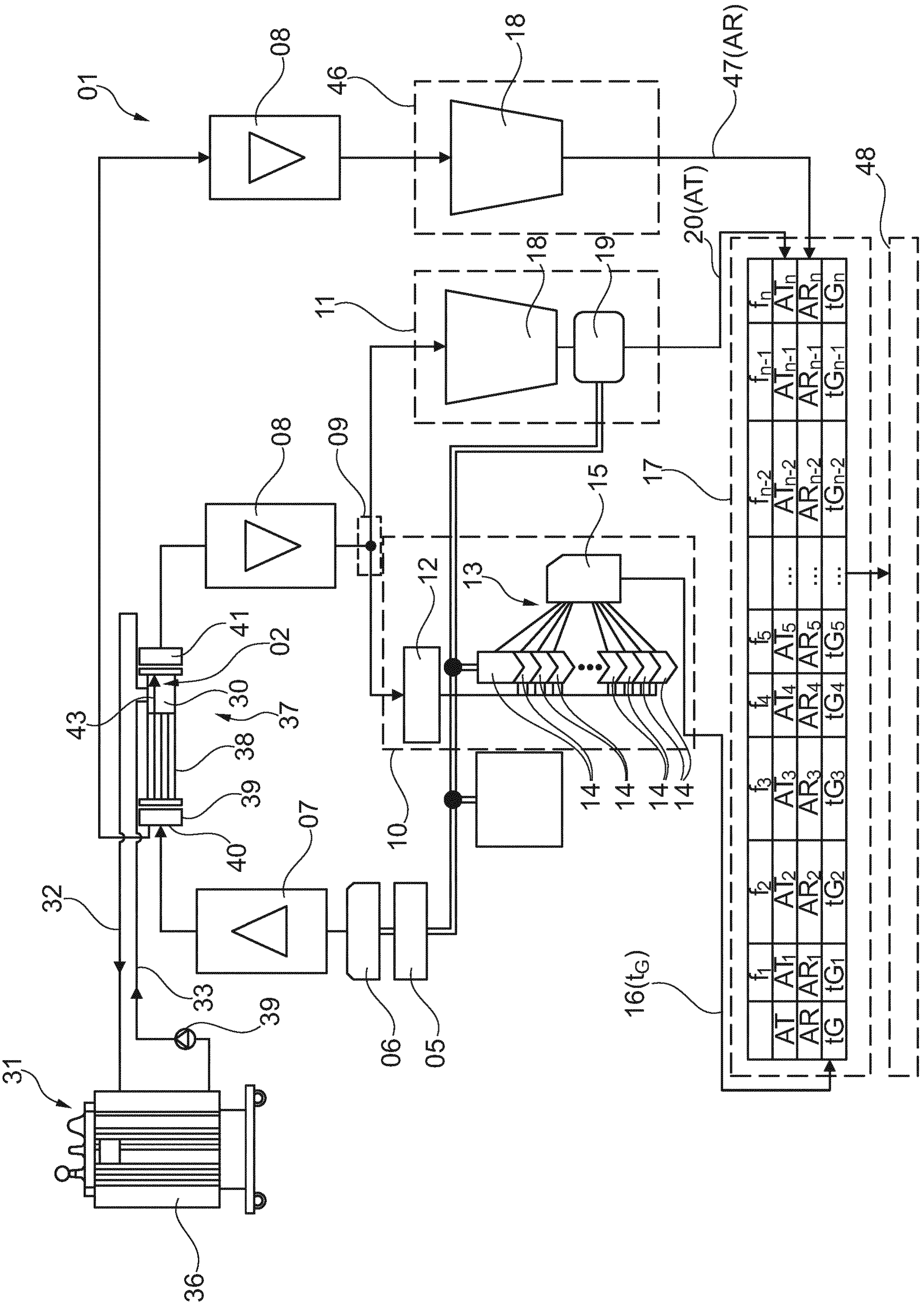
01
08
18
46
47(AR)
11
19
20(AT)
48
09
15
13
17
12
10
14
43
41
02
30
37
38
39
40
32
33
07
06
05
31
36
16(tG)
f1 f2 f3 f4 f5 ... fn-2 fn-1 fn
AT AT1 AT2 AT3 AT4 AT5 ... ATn-2 ATn-1 ATn
AR AR1 AR2 AR3 AR4 AR5 ... ARn-2 ARn-1 ARn
tG tG1 tG2 tG3 tG4 tG5 ... tGn-2 tGn-1 tGn

Fig. 1

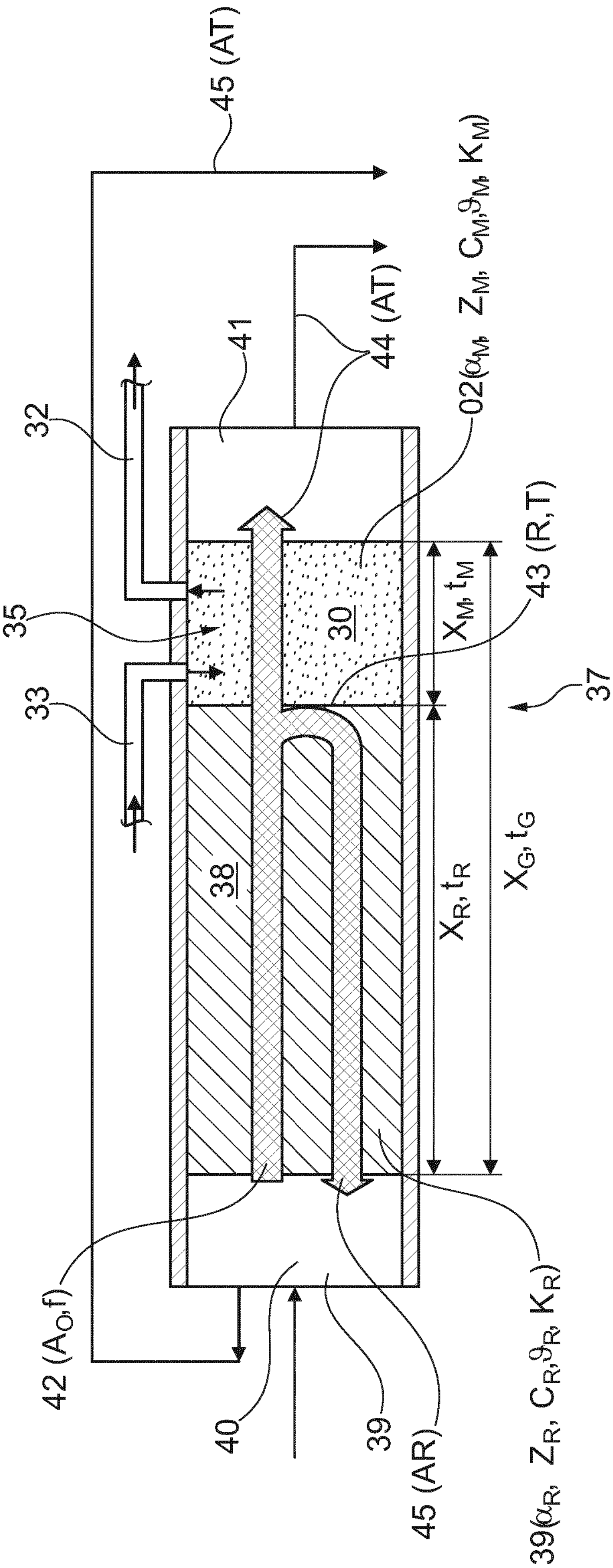
45 (AT)
44 (AT)
41
32
35
33
02(αM, ZM, CM, ϑM, KM)
43 (R,T)
30
38
XM, tM
XR, tR
XG, tG
37
42 (AO,f)
40
39
45 (AR)
39(αR, ZR, CR, ϑR, KR)

Fig. 2

| | |
|---|---|
| Schalldruck: | $P(x, t)) = P_{0} \cdot e^{-\alpha \cdot x} \cdot \sin(2\pi \ f \ (t - x/c))$ |
| Dämpfung: | $A(x) = A_{0} \cdot e^{-\alpha \cdot x}$ |
| Impedanz: | $Z = c \cdot p$ |
| Kompresibilität: | $K = Z \cdot c = c^{2} p$ |
| Reflexion und Transmission: | $R = \frac{Z_A - Z_B}{Z_A + Z_B}, \quad T = 1 - R = \frac{2 \cdot Z_B}{Z_A + Z_B}$ |

Fig. 3

48

$$Z_M = Z_R \cdot \frac{1-R}{1+R} = Z_R \cdot \frac{A_0 - A_R \cdot e^{2\alpha R \times R}}{A_0 + A_R \cdot e^{2\alpha R \times R}}$$

$$R = \frac{Z_R - Z_M}{Z_R + Z_M} \quad A = \frac{A_R \cdot e^{2\alpha R \times R}}{A_0}$$

$$\alpha_M = -\frac{1}{x_M} \cdot \ln \frac{A_2^{(T)} \cdot e^{2\alpha R \times R}}{A_0 \cdot T} = -\frac{1}{x_M} \cdot \ln \frac{A_T \cdot e^{2\alpha R \times R}}{A_0 \cdot (1-R)}$$

$$c_M = \frac{x_M}{t_M} = \frac{x_M}{t_G - t_R} = \frac{x_M}{t_G - \frac{x_G}{c_R}} = \frac{x_M}{t_G \cdot c_R - x_G} \cdot c_R$$

$$\rho_M = \frac{Z_M}{c_M} = \frac{Z_M(f)}{c_M(f)}$$

$$K_M = \rho_M \cdot c_M^2 = \begin{bmatrix} \rho_{M,1} \cdot c_{M,1}^2 \\ \rho_{M,2} \cdot c_{M,2}^2 \\ \vdots \\ \rho_{M,2} \cdot c_{M,H}^2 \end{bmatrix} = \begin{bmatrix} Z_{M,1} \cdot c_{M,1} \\ Z_{M,2} \cdot c_{M,2} \\ \vdots \\ Z_{M,2} \cdot c_{M,H} \end{bmatrix}$$

Fig. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 2006028096 A1
- DE 102008014300 A1
- DE 202007017911 U1
- US 5433112 A
- US 20150059442 A1
- DE 10324990 B2
- DE 19841154 A1
- DE 10324990 B3


### In der Beschreibung aufgeführte Nicht-Patentliteratur


- **JU BING-FENG et al.** Simultaneous measurement of longitudinal and transverse velocities, attenuation, density,. and thickness of films by using point-focus ultrasonic spectroscopy. *Journal of Applied Physics*
- **WERNER SCHAAFFS**. Molekularakustik. Springer Verlag, 1963
- **K.-H. HELLWEGE** ; **A.M. HELLWEGE** ; **W. SCHAAFFS**. Molecular Acustics/Molekularakustik. Springer Verlag, 1967
- **A.J. MATHESON**. Molecular Acustics. John Willy & Sons Verlag, 1971
- **JOSEF KRAUTKRÄMER** ; **HERBERT KRAUTKRÄMER**. Werkstoffprüfung mit Ultraschall. Springer-Verlag, 1975